# EUROPÄISCHE PATENTSCHRIFT

(11) **EP 1 150 820 B1**
(45) Veröffentlichungstag und Bekanntmachung des Hinweises auf die Patenterteilung: **24.03.2004**
(21) Anmeldenummer: 00987377.9
(22) Anmeldetag: 09.12.2000
(51) Int. Cl.: B29C 45/00

(54) **SPRITZGUSSVERFAHREN FÜR NEUTRALE UND SÄUREGRUPPENHALTIGE (METH)ACRYLAT-COPOLYMERE**
INJECTION MOLDING METHOD FOR NEUTRAL AND ACIDIC-GROUP CONTAINING (METH)ACRYLATE COPOLYMERS
PROCEDE DE MOULAGE PAR INJECTION POUR COPOLYMERES DE (METH)ACRYLATE NEUTRES ET CONTENANT DES GROUPES ACIDES

(30) Priorität: 17.12.1999 DE 19961334
(43) Veröffentlichungstag der Anmeldung: 07.11.2001
(73) Patentinhaber: Röhm GmbH & Co. KG, 64293 Darmstadt (DE)
(72) Erfinder: PETEREIT, Hans-Ulrich, 64291 Darmstadt (DE); BECKERT, Thomas, 64297 Darmstadt (DE); ASSMUS, Manfred, 64404 Bickenbach (DE); HÖSS, Werner, 63150 Heusenstamm (DE); FUCHS, Wolfgang, 64665 Alsbach (DE); SCHIKOWSKY, Hartmut, 64285 Darmstadt (DE)
(86) Internationale Anmeldenummer: PCT/EP2000/012467
(87) Internationale Veröffentlichungsnummer: WO 2001/043935

(56) Entgegenhaltungen:
- EP-A- 0 316 760
- EP-A- 0 364 060
- EP-A- 0 704 207
- DE-A- 19 646 432
- US-A- 4 738 817
- US-A- 5 531 736

## Beschreibung

Die Erfindung betrifft ein Verfahren zur Herstellung von Formkörpern mittels Spritzguß, die Formkörper selbst und deren Verwendung für pharmazeutische Zwecke.

### Stand der Technik

US 5 644 011 betrifft darmsaftlösliche Überzugs- und Bindemittel für Arzneiformen, enthaltend Copolymerisate aus 10 bis 25 Gew.-% Methacrylsäure, 40 bis 60 Gew.-% Methylacrylat und 20 bis 40 Gew.-% Methylmethacrylat. Die Anwendung erfolgt aus wäßriger Dispersion oder organischer Lösung.

EP 0 704 207 A2 beschreibt thermoplastische Kunststoffe für darmsaftlösliche Arzneiumhüllungen. Es handelt sich dabei um Mischpolymerisate aus 16 bis 40 Gew.-% Acryl- oder Methacrylsäure, 30 bis 80 Gew.-% Methylacrylat und 0 bis 40 Gew.-% anderen Alkylestern der Acrylsäure und/oder Methacrylsäure.

Die EP 0 704 207 A2 beschreibt beispielhaft Verfahren, bei denen verschiedene der oben genannten Mischpolymerisate zusammen mit dem Entformungshilfsmittel Glycerolmonostearat in Konzentrationen ab 1 und bis zu 6 Gew.-% zu Formkörpern in Kapselform spritzgegossen werden. In zwei Beispielen mit 5 und 6 Gew.-% Glycerolmonostearat ließen sich eine leichtere Entformbarkeit und und glattere Kapseloberflächen erreichen.

Im Beispiel werden entsprechende Mischpolymerisate bei 160 °C aufgeschmolzen und nach Zugabe von 6 Gew.-% Glycerinmonostearat gemischt. Die Mischung wird gebrochen und zu einem Pulver vermahlen. Das Pulver wird in die Vorkammer eines Spritzpreßwerkzeugs gefüllt und bei 170 °C unter einem Druck von 150 bar durch eine 0,5 mm weite Öffnung in den Formhohlraum gespritzt. Nach Abkühlung erhält man blasenfreie, leicht opake, dünnwandige Arzneimittelkapseln. Besondere Maßnahmen zur Entfernung niedrig siedender Bestandteile unmittelbar vor der Spritzgußverarbeitung sind nicht offenbart.

### Aufgabe und Lösung

Es wurde als Aufgabe gesehen, ein gegenüber der EP 0 704 207 A2 weiterentwickeltes Verfahren bereitzustellen, daß es erlaubt, neutrale oder anionische (Meth)acrylat-Copolymere im Spritzgußverfahren so zu verarbeiten, daß Spritzgießwerkzeugverunreinigungen möglichst nicht auftreten und zugleich hohe Ausbeuten bruch- und schlierenfreier Formkörper mit nur geringem Ausschuß erhalten werden können. Es sollen Formkörper erhalten werden, die hohen mechanischen Anforderungen genügen, maßhaltig sind um mit anderen Formkörpern einfach gefügt werden zu können und eine glatte, geschlossene Oberfläche ohne Poren und Riefen besitzen und sich daher als Träger oder als Behältnisse für pharmazeutische Wirkstoffe z. B. als Kapseln (Steckkapseln) oder Teile eignen.

Die Aufgabe wird gelöst durch ein Verfahren gemäß dem Anspruch 1
mit den Verfahrensschritten
A) Aufschmelzen einer Mischung aus
   a) einem (Meth)acrylat-Copolymeren, das sich aus 40 bis 100 Gew.-% radikalisch polymerisierten C₁- bis C₄-Alkylestern der Acryl- oder der Methacrylsäure und 0 bis 60 Gew.-% (Meth)acrylat-Monomeren mit einer anionischen Gruppe im Alkylrest zusammensetzt, und
   b) 0,1 bis 3 Gew.-% eines Trennmittels
      und gegebenenfalls
   c) 0 bis 50 Gew.-% eines Trockenstellmittels
   d) 0 bis 30 Gew.-% eines Weichmachers
   e) 0 bis 100 Gew.-% Additiven oder Hilfsstoffe
   f) 0 bis 100 Gew.-% eines pharmazeutischen Wirkstoffs
   g) 0 bis 20 Gew.-% eines weiteren Polymeren oder Copolymeren
   in der Mischung enthalten sein können, wobei sich die Mengenangaben der Komponenten b) bis g) auf das (Meth)acrylat-Copolymere a) beziehen und
   die Mischung vor dem Aufschmelzen einen Gehalt an niedrigsiedenden Bestandteilen mit einem Dampfdruck von mindestens 1,9 bar bei 120°C von über 0,5 Gew.-% aufweist,
B) Entgasen der Mischung im thermoplastischen Zustand bei Temperaturen von mindestens 120 °C, wodurch der Gehalt der niedrigsiedenden Bestandteile mit einem Dampfdruck von mindestens 1,9 bar bei 120°C auf höchstens 0,5 Gew.-% gesenkt wird
C) Einspritzen der aufgeschmolzenen und entgasten Mischung in den Formhohlraum eines Spritzgießwerkzeugs, wobei der Formhohlraum eine Temperatur aufweist, die mindestens 10 °C unterhalb der Glastemperatur des (Meth)acrylat-Copolymeren liegt, Abkühlen der Schmelzemischung und Entnahme des erhaltenen Formkörpers aus der Form.

Mittels des erfindungsgemäßen Verfahren sind neue spritzgegossene Formkörper erhältlich, die Anforderungen hinsichtlich hoher mechanischer Festigkeit, , maßhaltig sind um mit anderen Formkörpern einfach gefügt werden zu können und eine glatte, geschlossene Oberfläche ohne Poren und Riefen besitzen und hoher Temperaturstabilität genügen.

Die Erfindung umfaßt auch spritzgegossene Formkörper und deren Verwendung gemäß den jeweiligen Ansprüchen 5 und 8.

### Ausführung der Erfindung

Das erfindungsgemäße Verfahren zur Herstellung von Formkörpern mittels Spritzguß gliedert sich in die Verfahrensschritte A), B) und C).

Verfahrenschritt A) Aufschmelzen einer Mischung aus
a) einem (Meth)acrylat-Copolymeren, das sich aus 40 bis 100 Gew.-% radikalisch polymerisierten C₁- bis C₄-Alkylestern der Acryl- oder der Methacrylsäure und 0 bis 60 Gew.-% (Meth)acrylat-Monomeren mit einer anionischen Gruppe im Alkylrest zusammensetzt, das
b) 0,1 bis 3 Gew.-% eines Trennmittels enthält
   und gegebenenfalls
c) 0 bis 50 Gew.-% eines Trockenstellmittels
d) 0 bis 30 Gew.-% eines Weichmachers
e) 0 bis 100 Gew.-% Additive oder Hilfsstoffe
f) 0 bis 100 Gew.-% eines pharmazeutischen Wirkstoffs
g) 0 bis 20 Gew.-% eines weiteren Polymeren oder Copolymeren
in der Mischung enthalten sein können, wobei sich die Mengenangaben der Komponenten b) bis g) auf das (Meth)acrylat-Copolymere a) beziehen und
die Mischung vor dem Aufschmelzen einen Gehalt an niedrigsiedenden Bestandteilen mit einem Dampfdruck von mindestens 1,9 bar bei 120°C von über 0,5 Gew.-% aufweist,

Das Aufschmelzen des Copolymeren, das in Granulatform oder Pulverform vorliegt, erfolgt bevorzugt in einem Extruder bei einer Temperatur von 120°C bis 250 °C

### Die Mischung

Die Mischung besteht aus den Komponenten a) und b) sowie optional c) bis g)

### Das (Meth)acrylat-Copolymer a)

Das (Meth)acrylat-Copolymere besteht zu 40 bis 100, bevorzugt zu 45 bis 99, insbesondere zu 85 bis 95 Gew.-% aus radikalisch polymerisierten C₁- bis C₄-Alkylestern der Acryl- oder der Methacrylsäure und kann 0 bis 60, bevorzugt 1 bis 55, insbesondere 5 bis 15 Gew.-% (Meth)acrylat-Monomere mit einer anionischen Gruppe im Alkylrest enthalten.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomeren mit einer anionischen Gruppe im Alkylrest kann z. B. Acrylsäure, bevorzugt jedoch Methacrylsäure sein.

Geeignet sind z. B. neutrale (Meth)acrylat Copolymere aus 20 bis 40 Gew.-% Ethylacrylat und 60 bis 80 Gew.-% Methylmethacrylat (Typ EUDRAGIT® NE).

Weiterhin geeignet sind anionische (Meth)acrylat Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L100-55).

Ebenso geeignet sind anionische (Meth)acrylat Copolymere aus 20 bis 40 Gew.-% Methacrylsäure und 80 bis 60 Gew.-% Methylmethacrylat (Typ EUDRAGIT® S).

Besonders gut geeignet sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS).

Die Copolymere werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden.
Dies kann durch einfaches Brechen extrudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

### Trennmittel (Formtrennmittel) b)

Die Mischung enthält 0,1 bis 3, bevorzugt 0,2 bis 1 Gew.-% eines Trennmittels bezogen auf das (Meth)acrylat-Copolymere.

Im Gegensatz zu Trockenstellmitteln haben Formtrennmittel die Eigenschaft, die Klebkraft zwischen dem Formteilen und der Werkzeugwandung, durch die das Formteil hergestellt wird, zu reduzieren. Dadurch wird es möglich, Formteile herzustellen, die nicht zerbrochen und geometrisch bei der Entformung nicht deformiert werden. Formtrennmittel sind meist teilverträglich oder unverträglich mit den Polymeren, in denen sie besonders wirksam sind. Durch die Teil- bzw Unverträglichkeit tritt beim Einspritzen der Schmelze in den Formhohlraum eine Migration in die Grenzfläche des Überganges zwischen Werkzeugwandung und Formteil auf.

Damit Formtrennmittel besonders vorteilhaft migrieren können, muss der Schmelzpunkt des Formtrennmittels 20°C bis 100°C unterhalb der Verarbeitungstemperatur des Polymeren liegen.

Beispiele für Trennmittel (Formtrennmittel) sind:
Ester von Fettsäuren oder Fettsäureamide , aliphatische, langkettige Carbonsäuren, Fettalkohole sowie deren Ester, Montan- oder Paraffinwachse und Metallseifen, insbesondere zu nennen sind Glycerolmonostearat, Stearylalkohol, Glycerolbehensäureester, Cetylalkohol, Palmitinsäure, Kanaubawachs, Bienenwachs etc..

### Trockenstellmittel c)

Die Mischung kann 0 bis 50, bevorzugt 10 bis 30 Gew.-% eines Trockenstellmittels bezogen auf das (Meth)acrylat-Copolymere enthalten.

Trockenstellmittel haben folgende Eigenschaften: sie verfügen über große spezifische Oberflächen, sind chemisch inert, sind gut rieselfähig und feinteilig. Aufgrund dieser Eigenschaften lassen sie sich vorteilhaft in Schmelzen homogen verteilen und erniedrigen die Klebrigkeit von Polymeren, die als funktionelle Gruppen stark polare Comonomere enthalten.

Beispiele für Trockenstellmittel sind:
Aluminiumoxid, Magnesiumoxid, Kaolin, Talkum, Kieselsäure (Aerosile), Bariumsulfat, Ruß und Cellulose.

### Weichmacher d)

Die Mischung kann 0 bis 30, bevorzugt 0,5 bis 15 Gew.-% eines Weichmachers bezogen auf das (Meth)acrylat-Copolymere enthalten.

Der Zusatz von Weichmacher bewirkt eine geringere Sprödigkeit der Formkörper. Dadurch reduziert sich der Anteil gebrochener Formkörper nach Entformung. Ohne Weichmacher liegt der Anteil einwandfrei entnommener Formkörper bei den meisten Mischungen in der Regel bei etwa 85 %. Mit Weichmacherzusatz kann der Anteil an Entformungsbruch reduziert werden, so daß die Ausbeuten insgesamt meist auf 95 bis 100 % gesteigert werden können.

Als Weichmacher geeignete Stoffe haben in der Regel ein Molekulargewicht zwischen 100 und 20 000 und enthalten eine oder mehrere hydrophile Gruppen im Molekül, z. B. Hydroxyl- , Ester- oder Aminogruppen. Geeignet sind Citrate, Phthalate, Sebacate, Rizinusöl. Beispiele geeigneter Weichmacher sind Citronensäurealkylester, Glycerinester, Phtalsäurealkylester, Sebacinsäurealkylester, Sucroseester, Sorbitanester, Dibutylsebacat und Polyethylenglykole 400 g/mol bis 20.000 g/mol.

Bevorzugte Weichmacher sind Tributylcitrat, Triethylcitrat, Acetyltriethylcitrat, Dibutylsebacat und Diethylsebacat.

### Additive oder Hilfsstoffe e)

Die Mischung kann 0 bis 100 Gew.-% von in der pharmazeutisch üblichen Additiven oder Hilfsstoffe bezogen auf das (Meth)acrylat-Copolymere enthalten.

Hier sind z. B, Stabilisatoren, Farbstoffe, Antioxidantien, Netzmittel, Pigmente, Glanzmittel etc. zu nennen.

### Pharmazeutischer Wirkstoff f)

Die Mischung kann 0 bis 100 Gew.-% eines oder mehrerer pharmazeutischer Wirkstoffe bezogen auf das (Meth)acrylat-Copolymere enthalten. Es sollen dabei solche pharmazeutische Wirkstoffe eingesetzt werden, die sich bei Verarbeitungstemperatur nicht zersetzten.

Die im Sinne der Erfindung eingesetzten Arzneistoffe (pharmazeutischer Wirkstoffe) sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.

Erfindungsgemäß können alle Wirkstoffe eingesetzt werden, die die gewünschte therapeutische Wirkung im Sinne der obigen Definition erfüllen und eine ausreichendeStabilität sowie Penetrationsfähigkeit durch die Haut besitzen.

Wichtige Beispiele (Gruppen und Einzelsubstanzen) ohne Anspruch auf Vollständigkeit sind folgende:
Analgetika,
Antiallergika, Antiarrhythmika
Antibiotika, Chemotherapeutika, Antidiabetika, Antidote,
Antiepileptika, Antihypertonika, Antihypotonika,
Antikoagulantia, Antimykotika, Antiphlogistika,
Betarezeptorenblocker, Calciumantagonisten und ACE-Hemmer,
Broncholytika/Antiasthmatika, Cholinergika, Corticoide (Interna),
Dermatika, Diuretika, Enzyminhibitoren, Enzympräparate und Transportproteine,
Expectorantien, Geriatrika, Gichtmittel, Grippemittel,
Hormone und deren Hemmstoffe, Hypnotika/Sedativa, Kardiaka, Lipidsenker,
Nebenschilddrüsenhormone/Calciumstoffwechselregulatoren,
Psychopharmaka, Sexualhormone und ihre Hemmstoffe,
Spasmolytika, Sympatholytika, Sympathomimetika, Vitamine,
Wundbehandlungsmittel, Zytostatika.

Beispiele für zum Einfüllen in die Formkörper (Kapseln) oder auch für die Einarbeitung in die Formkörper geeignete Wirkstoffe sind: Ranitidin, Simvastatin, Enalapril, Fluoxetin, Amlodipin, Amoxicillin, Sertalin, Nifidipin, Ciprofloxacin, Acycolvir, Lovastatin,Epoetin, Paroxetin, Captopril, Nabumeton, Granisetron, Cimetidin, Ticarcillin, Triamteren, Hydrochlorothiazid, Verapamil, Paracetamol, Morphinderivate, Topotecan oder der pharmazeutisch verwendeten Salze.

### Weitere Polymere oder Copolymere g)

Die Mischung kann 0 bis 20 Gew.-% eines weiteren Polymeren oder Copolymeren bezogen auf auf das (Meth)acrylat-Copolymere enthalten.

Zur Steuerung der Wirkstoffabgabe kann es im Einzelfall vorteilhaft sein, weitere Polymere zuzumischen. Der Anteil weiterer Polymere an der Mischung beträgt jedoch nicht mehr als 20 Gew.-%, bevorzugt höchstens 10 Gew.-%, insbesondere 0 - 5 Gew.-%, bezogen auf das (Meth)acrylat-Copolymere.

Beispiele für solche weiteren Polymere sind: Polyvinylpyrolidone, Polyvinylalkohole, kationische (Meth)acrylat-Copolymere aus Methylmethacrylat und/oder Ethylacrylat und 2-Dimethylaminoethylmethacrylat (EUDRAGIT® E100), Carboxymethylcellulose-Salze, Hydroxypropylcellulose (HPMC), neutrale (Meth)acrylat Copolymere aus Methylmethacrylat und Ethylacrylat (Trockensubstanz aus EUDRAGIT® NE 30 D), Copolymere aus Methylmethacrylat und Butylmethacrylat (PLASTOID® B) oder (Meth)acrylat Copolymere mit quaternären Ammoniumgruppen,
Trimethylammoniumethylmethacrylat-Chlorid als Monomer enthaltend (EUDRAGIT® RL bzw. EUDRAGIT® RS).

### Niedrigsiedende Bestandteile

Das an sich bekannte (Meth)acrylat-Copolymer weist in seiner Handelsform praktisch immer einen Gehalt an niedrigsiedenden Bestandteilen mit einem Dampfdruck von mindestens 1,9 bar bei 120°C von über 0,5 Gew.-% auf. Übliche Gehalte dieser Bestandteile liegen im Bereich von 0,7 bis 2,0 Gew-%. Bei den niedrigsiedenden Bestandteilen handelt es sich in der Hauptsache um Wasser, das aus der Luftfeuchtigkeit aufgenommen wird oder aus dem Herstellprozeß der Polymeren mit enthalten ist.

### Verfahrensschritt B)

Entgasen der Mischung bei Temperaturen von mindestens 120 °C, bevorzugt mindestens 150 °C und höchstens 250 °C, wodurch der Gehalt der niedrigsiedenden Bestandteile mit einem Dampfdruck von mindestens 1,9 bar bei 120°C auf höchstens 0,5 Gew.-%, bevorzugt höchstens 0,2 Gew.-% , besonders bevorzugt höchstens 0,1 Gew.-% gesenkt wird. Dadurch kann vermieden werden, daß es während des Spritzgußvorganges in Verfahrensschritt c) zu einer unerwünschten plötzlichen Ausgasung kommt, die zu Blasenbildung oder einem Aufschäumen innerhalb des entstehenden Formkörpers führen würde, der dann unbrauchbar wäre.

Da die angeführten (Meth)acrylat-Copolymere entweder eine niedirge Glastemperatur aufweisen und deshalb schon bei niedrigen Temperaturen verkleben können oder aber thermisch labil sind, können niedrigsiedende Bestandteile in der Regel nicht durch einfaches Trocken bei erhöhter Temperatur entfernt werden.

Deshalb wird der Entgasungsschritt b) bevorzugt durch Extrusionstrocknung mittels eines Extruders mit Entgasungszone oder mittels einer Spritzgießanlage mit einem Spritzgießwerkzeug mit vorgeschalteter Entgasungsöffnung ausgeführt.

Zum effektiveren Betrieb der Entgasung kann auch die Installation einer vakuumerzeugenden Pumpe (z. B. Wasserstrahlpumpe) an der Entgasungsöffnung des Extruders oder der Spritzgießmaschine vorgenommen werden. Die damit erzeugbaren Unterdrücke führen zu einer weitgehenderen Entfernung der niedrigsiedenden Bestandteile, wie z.B. Feuchtigkeit aus der Schmelze. Dadurch erzeugbare Unterdrücke können von 800 mbar bis 10 mbar liegen.

Das durch Extrusionstrocknung in einem Extruder mit Entgasungszone erhaltene, entgaste Extrudat kann ohne weitere Verfahrensschritte zur Entfernung niedersiedender Bestandteile unmittelbar auf die Spritzgießmaschine gegeben und direkt zu Formkörpern verarbeitet werden.

Beim Entgasen auf einer Spritzgießanlage mit vorhandener Entgasungsöffnung im Spritzgießzylinder erfolgt die Entgasung vor dem Einpressen der Kunststoffschmelze in die Spritzgießform mittels der genannten Entgasungsöffnung im Spritzgießzylinder.

### Verfahrensschritt C)

Einspritzen der aufgeschmolzenen und entgasten Mischung in den Formhohlraum eines Spritzgießwerkzeugs, wobei der Formhohlraum eine Temperatur aufweist, die mindestens 10 °C, bevorzugt mindestens 12 °C, besonders bevorzugt mindestens 15 °C, insbesondere mindestens 25 °C oder sogar mindestens 35 °C unterhalb der Glastemperatur des (Meth)acrylat-Copolymeren liegt, Abkühlen der Schmelzemischung und Entnahme des erhaltenen Formkörpers aus der Form.

Die thermoplastische Verarbeitung erfolgt in an sich bekannter Weise mittels einer Spritzgußmaschine bei Temperaturen im Bereich von 80 bis 220 °C, insbesondere zwichen 120 °C und 160°C und bei Drücken von 60 bis 400bar.

Die Formtemperatur liegt bei Glastemperaturen der eingesetzten (Meth)acrylat-Copolymeren in Bereich von z. B. 40°C bis 80°C entsprechend niedriger z. B. bei höchstens 30 oder höchstens 20 °C, so daß das Copolymer bereits nach kurzer Zeit nach dem Einspritzvorgang in der Form erstarrt und der fertige Formkörper entnommen bzw. entformt werden kann.

Die Formkörper können aus der Formhöhlung des Spritzgießwerkzeuges ohne Bruch entformt werden und weisen eine gleichmäßige, kompakte einwandfreie Oberfläche auf. Der Formkörper zeichnet sich durch mechanische Belastbarkeit bzw. Elastizität und Bruchfestigkeit aus.

Er weist insbesondere eine Schlagzähigkeit nach ISO 179 gemessen an Probekörpern von mindestens 5 KJ/m², bevorzugt von mindestens 10 KJ/m², besonders bevorzugt von mindestens 15 KJ/m2 auf.

Die Wärmeformbeständigkeit VST (A10), gemessen an Probekörpern nach ISO 306 liegt in etwa zwischen 30°C und 60°C.

Die erfindungsgemäß erhaltenen Formkörper können z. B. die Form einer Kapsel, den Teil einer Kapsel, z. B. einer Kapselhälfte, oder einer Steckkapsel aufweisen, die als Behältnis für einen pharmazeutischen Wirkstoff dient. Es können Wirkstoffe z. B. in Form von Pellets eingefüllt werden und hiernach die beiden Kapselteile durch Kleben, Verschweißen durch Laser, Ultraschall bzw. Mikrowellen oder mittels Schnappverbindung zusammengefügt werden.

Nach diesem Verfahren können erfindungsgemäß auch Kapseln aus unterschiedlichem Material (z.B. Gelatine, anhydrolysierte Stärke, HPMC oder andere Methacrylate) miteinander kombiniert werden. Der Formkörper kann somit auch Teil einer Dosiereinheit sein.

Auch andere Formen, wie Tabletten- oder Linsengeometrien sind möglich. Hierbei enthält der Compound, der für das Spritzgießen zum Einsatz kommt bereits den pharmazeutischen Wirkstoff. In der endgültigen Form liegt der Wirkstoff möglichst gleichmäßig verteilt in kristalliner (Solid Dispersion) oder gelöster Form (Solid Solution) vor.

### BEISPIELE

### Beispiel 1: Darmsaftlöslicher Formkörper

In einen 30I Mischbehälter aus Edelstahl werden 10kg eines (Meth)acrylat Copolymers in Granulatform, bestehend aus Methylmethacrylat, Methylacrylat und Methacrylsäure in Verhältnis 25 : 65 : 10, gegeben und 12,5g Stearylalkohol (0.25 Gew-%) zugewogen und anschließend auf einem Taumelmischer 5min gemischt. Die hergestellte Mischung wurde auf einen Doppelschneckenextruder Leistritz LMS 30.34 gegeben um einen erfindungsgemäßen Compound herzustellen.
Die eingestellte Schmelzetemperatur betrug 180°C bei einer Schneckendrehzahl von 120U/min.

Nach einer Länge von 50% der Gesamtlänge des Doppelschneckenextruders ist in der Zylinderwandung eine Öffnung eingebracht, über die mittels einer Membranpumpe Triethylcitrat in einer Menge von 1 % bezogen auf die Polymermenge zugepumpt wird. Nach einer Mischzone für die Homogenisierung der Mischung ist im Schneckenzylinder eine Entgasungsöffnung eingebracht, die eine Öffnung zur Umgebung aufweist. Es kann beobachtet werden, dass aus der Entgasungszone Dampf austritt. Mittels einer Düse wurden vier Stränge aus dem Extruder abgeformt, über ein gekühltes Blech abgezogen und zu Granulat geschnitten. An erhaltenen Granulat wurde der Feuchtegehalt nach K. Fischer mit 0,08% ermittelt. Eine Überprüfung des nicht extrudierten Ausgangsgranulats ergab einen Wassergehalt von 1,2%.

### Spritzgießverarbeitung des erhaltenen Granulats:

Die so erhaltene, entgaste und granulierte Mischung wurde in den Trichter einer Spritzgießmaschine (Allrounder 250-125, Fa. Arburg) gegeben und zu Kapseln spritzgegossen.

Verwendet wurde ein Vierfach - Spritzgießwerkzeug mit Kaltkanal-Angußsystem. Die Kapseln haben einen Länge von 16mm, einen mittleren Außendurchmesser von 6,8mm, der sich zum geschlossenen Ende hin auf 4mm verjüngt und eine Wandstärke von 0,6mm.

An der Spritzgießmaschine wurden folgende Temperaturen eingestellt:
Zone 1 (Einzugszone): 70 °C, Zone 2: 160°C Zone 3: 160°C Zone 4: 160°C Zone 5 (Düse): 130°C
Einspritzdruck 60 bar, Nachdruck: 50 bar, Staudruck 3bar
Werkzeugtemperatur: 17°C

Nach dem Einspritzen der Schmelze und einer Nachdruckzeit von 6s wurde nach einer Kühlzeit von 18s das Werkzeug geöffnet und die Kapseln entformt. Die Formteile konnten ohne Bruch aus dem Spritzgießwerkzeug entformt werden. Es wurden transparente Kapseln erhalten die mechanisch stabil sind und für weitere Tests herangezogen werden können.

Nach dem Spritzgießen von 300 Schuß wurde der Zyklus unterbrochen um die Werkzeugoberfläche zu begutachten. Es konnte kein Belag festgestell werden. Die polierte Werkzeugoberfläche ist metallisch blank und hochglänzend.

### Beispiel 2: (Vergleichsbeispiel)

Ein Mischung wurde gemäß dem Beispiel der EP 0 704 207 A2 hergestellt. Statt den dort beschriebenen Copolymeren wurde ein (Meth)acrylat Copolymer in Granulatform, bestehend aus Methylmethacrylat, Methylacrylat und Methacrylsäure in Verhältnis 25 : 65 : 10 eingesetzt und gemäß EP 0 704 207 A2 mit 6 Gew-% Glycerolmonostearat vermischt.

Dazu wurden über eine gravimetrische Dosiereinrichtung in die Einzugszone des Doppelschneckenextruders 10 kg des (Meth)acrylat-Copolymeren und 600g Glycerolmonostearat kontinuierlich in die Einzugszone des Doppelschneckenextruders zudosiert.

Mit einer Schneckendrehzahl von 120U/min und einer Schmelzetemperatur von 160°C wurde die Komponenten im Extruder homogen in die Schmelze eingemischt.

Das Granulat wurde wie in Beispiel 1 auf die Spritzgußmachine gegeben und unter Beibehaltung der Parametereinstellung verarbeitet.
Nach 14 Spritzgießzyklen konnten Mattstellen auf den Oberflächen der hergestellten Kapseln festgestellt werden. Der Spritzgießzyklus wurde unterbrochen und das Spritzgießwerkzeug inspiziert. Auf den hochglanzpolierten Oberflächen der Werkzeugeinsätze konnte Belag festgestellt werden. Der Belag wurde mittels acetongetränkter Watte abgewischt und analysiert. Es konnte Glycerolmonostearat nachgewiesen werden.

### Beispiel 3: (Vergleichsbeispiel)

Es wurde gemäß Beispiel 1 eine Mischung (Compound) auf dem Doppelschneckenextruder hergestellt, wobei die Entgasungsöffnung am Ende des Extruders verschlossen war.

Am erhaltenen Granulat wurde der Feuchtegehalt nach K. Fischer mit 1,2% Wasser ermittelt.

Das erhaltene Granulat wurde wie in Beispiel 1 beschrieben auf die Spritzgußmaschine gegeben und verarbeitet. Die erhaltenen Kapseln zeigten Oberflächenfehler, wie Schlieren, Riefen und Unebenheiten auf und entsprechen nicht den gestellten Anforderungen.

## Patentansprüche

1. Verfahren zur Herstellung von **spritzgegossenen** Formkörpern
mit den Verfahrensschritten
A) Aufschmelzen einer Mischung aus
a) einem (Meth)acrylat-Copolymeren, das sich aus 40 bis 100 Gew.-% radikalisch polymerisierten C₁- bis C₄-Alkylestern der Acryl- oder der Methacrylsäure und 0 bis 60 Gew.-% (Meth)acrylat-Monomeren mit einer anionischen Gruppe im Alkylrest zusammensetzt, das und
b) 0,1 bis 3 Gew.-% eines Trennmittels
**wobei** gegebenenfalls
c) 0 bis 50 Gew.-% eines Trockenstellmittels
d) 0 bis 30 Gew.-% eines Weichmachers
e) 0 bis 100 Gew.-% Additive oder Hilfsstoffe
f) 0 bis 100 Gew.-% eines pharmazeutischen Wirkstoffs
g) 0 bis 20 Gew.-% eines weiteren Polymeren oder Copolymeren
in der Mischung enthalten sein können, wobei sich die Mengenangaben der Komponenten b) bis g) auf das (Meth)acrylat-Copolymere a) beziehen und
die Mischung vor dem Aufschmelzen einen Gehalt an niedrigsiedenden Bestandteilen mit einem Dampfdruck von mindestens 1,9 bar bei 120°C von über 0,5 Gew.-% aufweist,
B) Entgasen der Mischung im thermoplastischen Zustand bei Temperaturen von mindestens 120 °C, wodurch der Gehalt der niedrigsiedenden Bestandteile mit einem Dampfdruck von mindestens 1,9 bar bei 120°C auf höchstens 0,5 Gew.-% gesenkt wird
C) Einspritzen der aufgeschmolzenen und entgasten Mischung in den Formhohlraum eines Spritzgießwerkzeugs, wobei der Formhohlraum eine Temperatur aufweist, die mindestens 10 °C unterhalb der Glastemperatur des (Meth)acrylat-Copolymeren liegt, Abkühlen der Schmelzemischung und Entnahme des erhaltenen Formkörpers aus der Form.

2. Verfahren nach Anspruch 1, **dadurch gekennzeichnet, daß** der Entgasungsschritt b) durch Extrusionstrocknung mittels eines Extruders mit Entgasungszone oder mittels einer Spritzgießanlage mit einer, dem Spritzgießwerkzeug vorgeschalteten Entgasungsöffnung im Spritzgießzylinder erfolgt.

3. Verfahren nach Anspruch 1 oder 2, **dadurch gekennzeichnet**, das (Meth)acrylat-Copolymere als (Meth)acrylat-Monomer mit einer anionischen Gruppe im Alkylrest 1 bis 50 Gew.-% Methacrylsäure enthält.

4. Verfahren nach einem oder mehreren der Ansprüche 1 bis 3, **dadurch gekennzeichnet, daß** die Mischung 0,5 bis 25 Gew.-% Weichmacher enthält.

5. Spritzgegossener Formkörper aus einer Mischung aus den die Komponenten a) und b) sowie gegebenenfalls c) bis g) gemäß den in Anspruch 1 angegebenen Mengenanteilen und herstellbar in einem Verfahren gemäß Anspruch 1, bei dem der Gehalt der niedrigsiedenden Bestandteile in der Mischung mit einem Dampfdruck von mindestens 1,9 bar bei 120 °C auf höchstens 0,5 Gew.-% gesenkt wird, bevor die aufgeschmolzene und entgaste Mischung in den Formhohlraum eines Spritzgießwerkzeuges eingespritzt wird.

6. Formkörper nach Anspruch 5, **dadurch gekennzeichnet, daß** er eine Schlagzähigkeit nach ISO 179 von mindestens 5 KJ/m² aufweist.

7. Formkörper nach Anspruch 5 oder 6, **dadurch gekennzeichnet, daß** der Formkörper eine Kapsel, ein Teil einer Kapsel oder ein Teil einer Dosiereinheit ist.

8. Verwendung eines Formkörpers nach einem oder mehreren der Ansprüche 5 bis 7 als Behältnis oder als Träger für einen pharmazeutischen Wirkstoff.

## Claims

1. Process for preparing injection-moulded mouldings comprising the steps of
A) melting a mixture of
a) a (meth)acrylate copolymer made up of 40 to 100 wt.% of radically polymerised C₁-C₄ alkyl esters of acrylic or methacrylic acid and 0 to 60 wt.% of (meth)acrylate monomers with an anionic group in the alkyl moiety, and
b) 0.1 to 3 wt.% of a mould release agent,
the mixture optionally containing
c) 0 to 50 wt.% of a drying agent
d) 0 to 30 wt.% of a plasticiser
e) 0 to 100 wt.% of additives or adjuvants
f) 0 to 100 wt.% of a pharmaceutically active substance
g) 0 to 20 wt.% of another polymer or copolymer,
the quantities of components b) to g) specified being based on the (meth)acrylate copolymer a) and the mixture before melting having a content of more than 0.5 wt.% of low-boiling ingredients with a vapour pressure of at least 1.9 bar at 120°C,
B) degassing the mixture in the thermoplastic state at temperatures of at least 120°C, thereby reducing the content of low boiling ingredients having a vapour pressure of at least 1.9 bar at 120°C to not more than 0.5 wt.%,
C) injecting the molten degassed mixture into the moulding cavity of an injection moulding tool, the moulding cavity having a temperature which is at least 10°C below the glass transition temperature of the (meth)acrylate copolymer, cooling the molten mixture and removing the resulting moulding from the mould.

2. Process according to claim 1, **characterised in that** the degassing step b) is carried out by extrusion drying using an extruder with a degassing zone or by means of an injection moulding apparatus having a degassing vent in the injection moulding cylinder upstream of the injection moulding tool.

3. Process according to claim 1 or 2, **characterised in that** the (meth)acrylate copolymer contains 1 to 50 wt.% of methacrylic acid as the (meth)acrylate monomer having an anionic group in the alkyl moiety.

4. Process according to one or more of claims 1 to 3, **characterised in that** the mixture contains 0.5 to 25 wt.% of plasticiser.

5. Injection moulded moulding consisting of a mixture of components a) and b) and optionally c) to g) in the proportions specified in claim 1 and capable of being prepared by a process according to claim 1, wherein the content of low-boiling ingredients in the mixture having a vapour pressure of at least 1.9 bar at 120°C is reduced to not more than 0.5 wt.% before the molten degassed mixture is injected into the moulding cavity of an injection moulding tool.

6. Moulding according to claim 5, **characterised in that** it has a notching strength according to ISO 179 of at least 5 KJ/m².

7. Moulding according to claim 5 or 6, **characterised in that** the moulding is a capsule, part of a capsule or part of a dosage unit.

8. Use of a moulding according to one or more of claims 5 to 7 as a container or as a carrier for a pharmaceutically active substance.

## Revendications

1. Procédé de fabrication de corps moulés par injection sous pression, avec les étapes de procédé :
A) Fusion d'un mélange de :
a) un copolymère d'acrylate/de méthacrylate qui se compose de 40 à 100 % en masse d'esters du groupe alkyle C₁ à C₄ de polymérisation radicalaire de l'acide acrylique ou de l'acide méthacrylique et de 0 à 60 % en masse de monomères d'acrylate/de méthacrylate avec un groupe anionique dans le radical alcoyle, et
b) 0,1 à 3 % en masse d'un agent séparateur
dans lequel le cas échéant
c) 0 à 50 % en masse d'un agent siccatif
d) 0 à 30 % en masse d'un fluidifiant
e) 0 à 100 % en masse d'additifs ou de matières auxiliaires
f) 0 à 100 % en masse d'un principe actif pharmaceutique
g) 0 à 20 % en masse d'un autre polymère ou copolymère
peuvent être contenus dans le mélange, les indications de quantité des composants b) à g) se rapportant au copolymère d'acrylate/de méthacrylate a) et le mélange présentant, avant fusion, une teneur en constituants à bas point d'ébullition de plus de 0,5 % en masse avec une pression de vapeur d'au moins 1,9 bars à 120°C,
B) Dégazage du mélange à l'état thermoplastique à des températures d'au moins 120°C, par lequel la teneur des constituants à bas point d'ébullition est abaissée à au plus 0,5 % en masse avec une pression de vapeur d'au moins 1,9 bars à 120°C,
C) Injection du mélange fondu et dégazé dans l'empreinte d'un moule à injection, l'empreinte présentant une température qui est au moins de 10°C en dessous de la température de vitrification du copolymère d'acrylate/de méthacrylate, refroidissement du mélange fondu et extraction du corps moulé obtenu hors du moule.

2. Procédé selon la revendication 1,
**caractérisé en ce que**
l'étape de dégazage b) est réalisée dans le cylindre d'injection par un séchage à extrusion au moyen d'une extrudeuse avec zone dégazeuse ou au moyen d'un dispositif d'injection avec un orifice de dégazage raccordé au moule à injection.

3. Procédé selon la revendication 1 ou 2,
**caractérisé en ce que**
le copolymère d'acrylate/de méthacrylate comme le monomère d'acrylate/de méthacrylate avec un groupe anionique dans le radical alcoyle contient 1 à 50 % en masse d'acide méthacrylique.

4. Procédé selon l'une ou plusieurs des revendications 1 à 3,
**caractérisé en ce que**
le mélange contient 0,5 à 25 % en masse de fluidifiant.

5. Corps moulé par injection à partir d'un mélange avec des composants a) et b) et, le cas échéant c) à g), selon les quantités indiquées en revendication 1 et fabricable par un procédé selon la revendication 1, dans lequel la teneur des constituants à bas point d'ébullition est abaissée au maximum 0,5 % en masse dans le mélange, avec une pression de vapeur d'au moins 1,9 bars à 120°C, avant que le mélange fondu et dégazé soit injecté dans l'empreinte d'un moule à injection.

6. Corps moulé selon la revendication 5,
**caractérisé en ce qu'**
il contient une résilience selon la norme ISO 179 d'au moins 5 KJ/m².

7. Corps moulé selon la revendication 5 ou 6,
**caractérisé en ce que**
le corps moulé est une capsule, une partie d'une capsule ou une partie d'une unité de dosage.

8. Utilisation d'un corps moulé selon l'une ou plusieurs des revendications 5 à 7, comme contenant ou comme substance vectrice pour un principe actif pharmaceutique.
